(19) Europäisches Patentamt

European Patent Office

Office européen des brevets

(11) **EP 1 693 462 A1**

(12) **EUROPEAN PATENT APPLICATION**
published in accordance with Art. 158(3) EPC

(43) Date of publication:
**23.08.2006 Bulletin 2006/34**

(51) Int Cl.:
*C12Q 1/26* (2006.01)      *C12Q 1/37* (2006.01)
*G01N 33/72* (2006.01)

(21) Application number: **04818961.7**

(22) Date of filing: **18.11.2004**

(86) International application number:
**PCT/JP2004/017196**

(87) International publication number:
**WO 2005/049858 (02.06.2005 Gazette 2005/22)**

(84) Designated Contracting States:
**AT BE BG CH CY CZ DE DK EE ES FI FR GB GR
HU IE IS IT LI LU MC NL PL PT RO SE SI SK TR**

(30) Priority: **19.11.2003 JP 2003389930**

(71) Applicant: **DAIICHI PURE CHEMICALS CO., LTD.**
**Chuo-ku**
**Tokyo 103-0027 (JP)**

(72) Inventors:
• **TANIGUCHI, Yuriko,**
**Daiichi Pure Chem. Co., Ltd.**
**Ryugasaki-shi,**
**Ibaraki 3010852 (JP)**

• **SAITO, Kazunori,**
**Daiichi Pure Chem. Co., Ltd.**
**Ryugasaki-shi,**
**Ibaraki 3010852 (JP)**

(74) Representative: **Hartz, Nikolai**
**Wächtershauser & Hartz**
**Weinstrasse 8**
**80333 München (DE)**

(54) **METHOD OF DETERMINING SUBSTRATE CONTAINED IN HEMOGLOBIN-CONTAINING SAMPLE**

(57)      The present invention provides a convenient, efficient method for determining a substrate contained in a hemoglobin-containing sample and a reagent therefor, which can be employed for a variety of automatic analyzers while reducing interference of hemoglobin contained in the sample.

A method for determining a substrate contained in a hemoglobin-containing sample through reaction of an oxidase with the substrate and optical measurement of the produced hydrogen peroxide by use of a peroxidase and an oxidizable color producing reagent, **characterized in that** the hemoglobin-containing sample is treated with an anionic surfactant selected from among a polyoxyethylene alkyl ether sulfate salt, a polyoxyethylene alkylphenyl ether sulfate salt, a polyoxyethylene alkyl ether phosphate, a polyoxyethylene alkyl sulfosuccinate, a polyoxyethylene alkyl ether carboxylate salt, a polyoxyethylene alkyl ether sulfonate salt, triethanolamine lauryl sulfate, an alkyl sulfosuccinate, and an alkylphenyl ether sulfonate salt.

EP 1 693 462 A1

**Description**

Technical Field

The present invention relates to a method for determining a substrate contained in a hemoglobin-containing sample through an enzyme reaction. Specifically, this invention relates to a method of reducing interference attributed to the hemoglobins coexisting in a sample and thereby allowing for accurate measurement of the substrate, and also to a reagent to be used for said method.

Background Art

As relationship between a disease and the quantity change of a specific component existing in a biological sample (e.g., blood or urine) is elucidated increasingly, the measurement of such a component has more and more become essential in diagnosis of a disease, elucidation of pathological conditions, judgment of a therapeutic effect, etc. The currently common method for determining a specific component contained in a biological sample (e.g., serum) is represented by an enzyme method in which the component of interest or a substance derived from the component of interest (hereinafter may be collectively referred to as "substrate") is reacted with an enzyme which acts specifically on the substrate, and the product is quantified. Among various enzyme methods, there is a method in which a hydrogen-peroxide-producing enzyme (hereinafter may be referred to as "oxidase enzyme") such as oxidase is reacted with a substrate, the produced hydrogen peroxide is reacted with a peroxidase and a color-developing agent which develops color when oxidized (hereinafter referred to as "oxidizable color producing reagent") to develop color, and then quantified by colorimetric analysis.

Examples of the substrate which is employed in the above method include glucose, cholesterol, neutral fat, phospholipid, free fatty acid, uric acid, creatinine, sialic acid, polyamine, and glycated hemoglobin. These substrates are actually measured in the clinical field.

However, the above method, which is based on oxidation reaction by an oxidase enzyme, is susceptible to interference by biological reducing substances coexisting in a sample (such as ascorbic acid, bilirubin, or hemoglobin), and therefore a negative error is inevitable. Furthermore, the following are also known. Bilirubin and hemoglobin are optically absorbed in the visible region and their wavelengths overlap the measurement wavelength of the colorimetric analysis, thus resulting in error. Moreover, absorption of bilirubin and hemoglobin changes over time under the influence of external light or a component contained in a reagent, which affects the results of the measurement. Besides, the above method tends to be adversely affected by turbidity attributed to lipid etc.

In normal cases, the amount of hemoglobin interfusing in a biological sample (serum, plasma, urine, saliva, cerebrospinal fluid, etc.) is very small. However, in the case of a disease involving hemolysis, or depending upon conditions for collecting blood or a similar sample or preparation procedure or storage conditions for a blood sample, hemoglobin may contaminate the sample through a secondary route. In addition, a whole blood sample, a hemocyte sample, or a sample prepared from whole blood or hemocytes through hemolysis naturally contains hemoglobin. Therefore, in a method for determining a substrate by use of an oxidase enzyme, reduction in interference of hemoglobin has become an important problem to be solved.

There has been known methods for reducing interference of hemoglobin in quantification of a substrate contained in a sample through use of an oxidase enzyme. For example, measurement wavelength is selected so as not to include absorption wavelength of hemoglobin itself (see, for example, Patent Document 1), or a cation or ampholytic surfactant is employed (see, for example, Patent Document 2).

However, in the method in which measurement wavelength is selected so as not to include absorption wavelength of hemoglobin itself, detection wavelength in colorimetric analysis is limited, and a measurement apparatus is also limited. In the method employing a cation or ampholytic surfactant, the surfactant may cause turbidity when mixed with a sample, or may affect enzyme activity.

Besides, a method employing two anionic surfactants in combination has also been known (see Patent Document 3). This method requires simultaneous use of a certain amount of an alkylsulfonate salt and an alkylnaphthalene sulfonate salt, to prevent denaturation of an enzyme protein caused by the anionic surfactants.

[Patent Document 1] JP-A-1997-119932
[Patent Document 2] JP-A-1991-10696
[Patent Document 3] JP-A-1996-89288

Disclosure of the Invention

Problems to be Solved by the Invention

The present invention provides a convenient, efficient method for determining a substrate contained in a hemoglobin-containing sample through an enzyme reaction, which can be employed for a variety of automatic analyzers while reducing interference of hemoglobin coexisting in the sample, and a reagent therefor.

Means for Solving the problem

In view of the forgoing, the present inventors have performed extensive studies for, in a method for determining a substrate in a sample through an enzyme reaction, reducing interference of hemoglobin coexisting in the sample in the measurement system, and found that interference of hemoglobin in the measurement system can be reduced through treatment of the sample with a certain anionic surfactant, accomplishing the present invention.

The present invention provides a method for determining a substrate contained in a hemoglobin-containing sample through reaction of an oxidase with the substrate and optical measurement of the produced hydrogen peroxide through use of a peroxidase and an oxidizable color producing reagent, characterized in that the hemoglobin-containing sample is treated with an anionic surfactant selected from among a polyoxyethylene alkyl ether sulfate salt, a polyoxyethylene alkylphenyl ether sulfate salt, a polyoxyethylene alkyl ether phosphate, a polyoxyethylene alkyl sulfosuccinate, a polyoxyethylene alkyl ether carboxylate salt, a polyoxyethylene alkyl ether sulfonate salt, triethanolamine lauryl sulfate, an alkyl sulfosuccinate, and an alkylphenyl ether sulfonate salt.

The present invention also provides a reagent for detecting a substrate contained in a hemoglobin-containing sample, containing (A) an anionic surfactant selected from among a polyoxyethylene alkyl ether sulfate salt, a polyoxyethylene alkylphenyl ether sulfate salt, a polyoxyethylene alkyl ether phosphate, a polyoxyethylene alkyl sulfosuccinate, a polyoxyethylene alkyl ether carboxylate salt, a polyoxyethylene alkyl ether sulfonate salt, triethanolamine lauryl sulfate, an alkyl sulfosuccinate, and an alkylphenyl ether sulfonate salt, (B) an oxidase which acts on the substrate to produce hydrogen peroxide, and (C) a peroxidase and an oxidizable color producing reagent.

The present invention also provides a method for determining glycated protein concentration, or glycated peptide or glycated amino acid concentration, or ratio of glycated peptide or glycated amino acid concentration to glycated protein concentration, characterized by employing at least (1) a surfactant, (2) a protease which acts on glycated protein to release fructosyl peptide, and (3) an enzyme which acts on fructosyl peptide to produce hydrogen peroxide.

The present invention also provides a method for determining hemoglobin concentration, hemoglobin A1c concentration, or ratio of hemoglobin A1c concentration to hemoglobin concentration of a hemoglobin-containing sample, characterized by comprising at least determining hemoglobin contained in the sample which has been treated with a surfactant, reacting a protease which releases fructosyl valylhistidine with the reaction mixture employed in the hemoglobin measurement, and determining hemoglobin A1c concentration of the mixture.

The present invention also provides a method for determining hemoglobin concentration, hemoglobin A1c concentration, or ratio of hemoglobin A1c concentration to hemoglobin concentration of a hemoglobin-containing sample, characterized by comprising a step of mixing the sample containing hemocytes with a reaction mixture containing a surfactant to thereby release hemoglobin from the hemocytes through hemolysis, a step of diluting the resultant mixture and optically determining hemoglobin concentration of the diluted mixture, a step of causing a protease to act on hemoglobin contained in the mixture to thereby release at least fructosyl valylhistidine, a step of causing an enzyme which reacts with fructosyl valylhistidine to thereby produce hydrogen peroxide to act at least on the released fructosyl valylhistidine, a step of reacting the produced hydrogen peroxide with a peroxidase and an oxidizable color producing reagent, a step of measuring change in absorbance of the colored compound to thereby determine the hemoglobin A1c concentration, and a step of calculating ratio of hemoglobin A1c concentration to hemoglobin concentration of the sample by use of the hemoglobin concentration and the hemoglobin A1c concentration.

The present invention also provides a method for pretreating a sample in measurement of hemoglobin concentration, hemoglobin A1c concentration, or ratio of hemoglobin A1c concentration to hemoglobin concentration, characterized by employing a nonionic surfactant and/or an anionic surfactant selected from among a polyoxyethylene derivative, a polyoxyethylene alkyl ether sulfate salt, a polyoxyethylene alkyl ether phosphate ester, triethanolamine lauryl sulfate, an alkyl sulfosuccinate, and an alkylphenyl ether sulfonate.

The present invention also provides a method for determining hemoglobin concentration of a sample including the steps of releasing hemoglobin from hemocytes through hemolysis and determining hemoglobin concentration, characterized by employing at least a nonionic surfactant and/or an anionic surfactant selected from among a polyoxyethylene derivative, a polyoxyethylene alkyl ether sulfate salt, a polyoxyethylene alkyl ether phosphate ester, triethanolamine lauryl sulfate, an alkyl sulfosuccinate, and an alkylphenyl ether sulfonate.

The present invention also provides a method for determining ratio of hemoglobin A1c concentration to hemoglobin concentration of a sample by means of a biochemical automatic analyzer, characterized in that, in setting operation conditions of the biochemical automatic analyzer, (1) operation conditions for measurement of hemoglobin concentration and for measurement of hemoglobin A1c concentration are individually set, (2) a reagent for determining hemoglobin concentration can also be used as a component of a reagent for determining hemoglobin A1c concentration, (3) a sample for hemoglobin concentration measurement and a sample for hemoglobin A1c concentration measurement can be used in common, and (4) the same wavelength can be employed in hemoglobin concentration measurement and hemoglobin A1c concentration measurement.

Effects of the Invention

The method for determining a substrate according to the present invention enables accurate measurement of a substrate contained in a sample while reducing interference of hemoglobin coexisting in the sample. The method for determining a substrate according to the present invention can be performed through a simple operation and thus can be applied to various analysis methods. Therefore, the method of the present invention is very useful in clinical tests.

Best Mode for Carrying Out the Invention

The method for determining a substrate contained in a sample according to the present invention may be performed in accordance with the known enzyme method, except that the sample is treated with a certain anionic surfactant for the purpose of reducing interference of hemoglobin which coexists in the sample.
No particular limitation is imposed on the sample employed in the present invention, so long as the sample contains hemoglobin. Examples of such a biological sample include whole blood, hemocytes, serum, plasma, cerebrospinal fluid, sweat, urine, lachrymal fluid, saliva, skin, mucosa, and hair. Of these, whole blood, hemocytes, serum, and plasma are preferred. The sample may be measured as it is; i.e., without undergoing any treatment. Alternatively, prior to the measurement, the sample may be filtered or dialyzed, or, if needed, the sample (substrate) may be subjected to concentration, extraction, dilution, or a similar process.
The dilution may be carried out using water or a buffer. No particular limitation is imposed on the type and the concentration of the buffer. Examples of the buffer include phosphate, phthalate, citrate, Tris, maleate, succinate, oxalate, tartrate, acetate, and Good's buffers (MES, PIPES, ADA, etc.). The buffer may be added at a concentration of 0.00001 to 2 mol/L, preferably 0.001 to 1 mol/L.
The anionic surfactant which may be employed in the method for determining a substrate according to the present invention is selected from among polyoxyethylene alkyl ether sulfate salts, polyoxyethylene alkylphenyl ether sulfate salts, polyoxyethylene alkyl ether phosphates, polyoxyethylene alkyl sulfosuccinates, polyoxyethylene alkyl ether carboxylate salts, polyoxyethylene alkyl ether sulfonate salts, triethanolamine lauryl sulfate, alkyl sulfosuccinates, and alkylphenyl ether sulfonate salts. The anionic surfactant is preferably a polyoxyethylene alkyl ether sulfate salt, a polyoxyethylene alkylphenyl ether sulfate salt, a polyoxyethylene alkyl ether phosphate, or an alkyl sulfosuccinate, particularly preferably a polyoxyethylene alkyl ether phosphate, a polyoxyethylene alkyl ether sulfate salt, or an alkyl sulfosuccinate, most preferably a polyoxyethylene alkyl ether phosphate. The anionic surfactant may be commercially available.
Examples of the polyoxyethylene alkyl ether sulfate salt include Nikkol SBL-4N, and Nikkol SBL-2T-36 (products of NOF Corporation); Emal 20T, Emal 327, and Emal 20C (products of Kao Corporation); Hitenol 225L, Hitenol 325D, Hitenol NF13, and Hitenol NF15 (products of Dai-ichi Kogyo Seiyaku Co., Ltd.); Sunnol LMT-1430, and Sunnol DM1470 (products of Lion Corporation). Examples of the polyoxyethylene alkylphenyl ether sulfate salt include Nikkol SNP-4N (product of NOF Corporation) and Emal NC35 (product of Kao Corporation). The polyoxyethylene alkyl ether phosphate may be a phosphoric acid monoester, a phosphoric acid diester, or a mixture of the monoester and the diester. Examples of the polyoxyethylene alkyl ether phosphate include Plysurf A208B, Plysurf A219B, Plysurf A208S, Plysurf A212C, Plysurf A212E, and Plysurf A215C (products of Dai-ichi Kogyo Seiyaku Co., Ltd.). Of these polyoxyethylene alkyl ether phosphate products, Plysurf A208B is preferred. Examples of the polyoxyethylene alkyl sulfosuccinate include Neo-Hitenol S-70, Neo-Hitenol L-30, and Neo-Hitenol LM-20 (products of Dai-ichi Kogyo Seiyaku Co., Ltd.). Examples of the polyoxyethylene alkyl ether carboxylate salt include Kao Akypo RLM-100NV (product of Kao Corporation), Neo-Hitenol ECL-30 (product of Dai-ichi Kogyo Seiyaku Co., Ltd.), Njcoap 2PS30, and Njcoap 2PS45 (products of New Japan Chemical Co., Ltd.). Examples of the polyoxyethylene alkyl ether sulfonate include Lionol OAI-N and Lionol OBI (products of Lion Corporation).
Examples of the triethanolamine lauryl sulfate include Emal TD (product of Kao Corporation). Examples of the alkyl sulfosuccinate include Pelex CS (product of Kao Corporation). Examples of the alkylphenyl ether sulfonate salt include Pelex SS-H (product of Kao Corporation).
The hemoglobin-containing sample may be treated through mixing of the sample with at least one of the anionic surfactants. The amount of the anionic surfactant employed is 0.0001 to 10%, preferably 0.001 to 3%, with respect to the mixture of the anionic surfactant and the sample such as whole blood, hemocytes, serum, etc. No particular limitation is imposed on the treatment time and the treatment temperature of the hemoglobin-containing sample. For example, when the sample is analyzed with an automatic analyzer, the sample is preferably treated for 5 minutes at 37°C. Prior to analysis with an automatic analyzer, the sample may be further treated. Conditions of these treatments may be suitably determined through experiment. No particular limitation is imposed on the pH conditions, additives, and the like to be employed when the sample is treated with an anionic surfactant, so long as the conditions and the additives have no adverse effect on the measurement of a substrate through enzyme reaction.

No particular limitation is imposed on the substrate to be determined through the method of the present invention, so long as the substrate can be measured through an enzyme reaction making use of an oxidase. Therefore, in the present invention, the "substrate" encompasses a substance which can be a substrate of an oxidase, and a substance which is produced through an enzyme reaction or a treatment and which can be a substrate of an oxidase (the substance derived from the component of interest, described above). Examples of the substrate include saccharide such as glucose, mannose, and galactose; lipids such as cholesterol, neutral fat, phospholipid, and free fatty acid; glycated proteins such as glycated albumin and glycated hemoglobin; uric acid, urea, creatinine, sialic acid, and polyamine.

Of these, glucose, uric acid, and the like correspond to the "substance which can be a substrate of an oxidase." Cholesterol which is obtained through treatment of an ester-type cholesterol contained in serum with a hydrolase, glycated peptide or glycated amino acid which is obtained through treatment of a glycated protein contained in serum with a protease, and the like correspond to the "substance which is produced through an enzyme reaction or a treatment and which can serve as a substrate of an oxidase."

As an example of the "substances which is produced through an enzyme reaction or a treatment and which can serve as a substrate of an oxidase," a glycated peptide or glycated amino acid which is obtained from a glycated protein through treatment with a protease and which serves as a substrate of an oxidase will next be described.

No particular limitation is imposed on the protease, so long as the protease has proteolysis or peptidolysis activity, and the protease may be derived from a microorganism, an animal, a plant, or the like. There is employed a protease which efficiently releases in a short period of time, from a target glycated protein (e.g., hemoglobin A1c), a glycated peptide or glycated amino acid, preferably fructosyl peptide or fructosyl amino acid, particularly preferably fructosyl valylhistidine or fructosyl valine. Specific examples of the protease include commercially available reagents for study purposes, such as proteinase K, trypsin, bromelain, carboxypeptidase, papain, pepsin, and aminopeptidase; and commercially available reagents for industrial use such as Neutral proteinase, and Toyozyme NEP (products of Toyobo, Ltd.); acid protease, alkaline protease, Molsin, AO protease, and peptidase (products of Kikkoman Corporation); Sumizyme CP, Sumizyme TP, and Sumizyme LP50D (products of Shin Nihon Chemical Co., Ltd.); Thermoase PC10F, Protin PC, Protin PC10F, Protin PS10, Protin NY10, Protin NL10, and Protin NC25 (products of Daiwa Kasei K.K.); Actinase AS (product of Kaken Pharmaceutical Co., Ltd.); Pronase E (product of Roche); Umamizyme, Protease S "Amano" G, Protease A "Amano" G, and Protease P "Amano" 3G (products of Amano Enzyme Inc.). Effect of the protease may be determined in the following manner: the protease is caused to act on the glycated protein or fructosyl peptide of interest, the glycated protein or fructosyl peptide before reaction and resultant product after reaction are subjected to capillary electrophoresis, and the results are analysed and compared between before and after the reaction with the protease. The protease may be employed singly or in combination of two or more species. The protease is preferably derived from a microorganism belonging to the genus *Bacillus, Aspergillus,* or *Streptomyces;* or produced from a gene of such a microorganism; or belongs to metalloproteinase, neutral protease, acid protease or basic protease.

No particular limitation is imposed on the concentration of the protease, so long as the protease can efficiently release a free substrate of interest at the concentration. An appropriate concentration of the protease may be experimentally determined in consideration of the specific activity or a similar factor of the protease. Adjustment of pH for the protease treatment is not necessarily performed, but the pH may be adjusted to a value which is suitable for the enzyme to act, within a range of 3 to 11. Adjustment of pH is carried out through use of an appropriate pH regulating agent such as a buffer. The treatment temperature is preferably 10 to 40°C.

In the method for determining a substrate according to the present invention, it may be allowed to use a known oxidase which is capable of oxidizing the substrate to be measured to thereby produce hydrogen peroxide. Examples include glucose oxidase, galactose oxidase, uricase, cholesterol oxidase, fructosyl amino acid oxidase (JP-A-2003-79386 and WO97/20039 pamphlet), ketoamine oxidase (JP-A-1993-19219), and fructosyl peptide oxidase (JP-A-2001-95598 and JP-A-2003-235585). The enzyme may be derived from a microorganism, an animal, a plant, or the like, or may be genetically engineered. In addition, the enzyme may or may not be chemically modified. The enzyme may be in solution form or dry form, and may be carried on or bound to an insoluble carrier. The enzyme may be used singly or in combination of two or more species.

The amount of the enzyme employed differs depending on the type of the enzyme, and may be experimentally determined appropriately in consideration of the specific activity of the enzyme etc. The enzyme is employed preferably in an amount of 0.001 to 1,000 U/mL, particularly preferably 0.01 to 1,000 U/mL. The pH conditions under which the enzyme is caused to act are adjusted by use of a buffer in consideration of the optimal pH of the enzyme. The reaction temperature may be appropriately selected from a temperature range which is ordinarily employed in enzyme reactions;

e.g., 10 to 40°C.

The oxidase may be used in combination with another enzyme, a coenzyme, an oxidizable color producing reagent, or the like, in accordance with needs. Examples of the enzyme include peroxidases, diaphorases, and amino acid-metabolizing enzymes which do not act on a substrate, fructosyl valine. Alternatively, an enzyme such as ascorbic acid oxidase or bilirubin oxidase may be employed for the purpose of treating interfering substances other than hemoglobin present in a sample. Examples of the coenzyme include nicotinamide adenine dinucleotide (NAD), reduced nicotinamide adenine dinucleotide (NADH), nicotinamide adenine dinucleotide phosphate (NADP), reduced nicotinamide adenine dinucleotide phosphate (NADPH), thio-NAD, and thio-NADP.

No particular limitation is imposed on the oxidizable color producing reagent, so long as the oxidizable color producing reagent is reacted with hydrogen peroxide to thereby develop color. Examples include a combination of 4-aminoantipyrine and a phenol compound, a naphthol compound, or an aniline compound, and a combination of 3-methyl-2-benzothiazolinonehydrazone and an aniline compound. Examples of the phenol compound which may be combined with 4-aminoantipyrine include phenol, p-chlorophenol, 2,4-dichlorophenol, 2,4-dibromophenol, and 2,4,6-trichlorophenol. Examples of the aniline compound which may be combined with 4-aminoantipyrine include N,N-dimethylaniline, N,N-diethylaniline, N,N-dimethyl-m-toluidine, N,N-diethyl-m-toluidine, N-ethyl-N-sulfopropyl-m-toluidine, N-ethyl-N-(2-hydroxy-3-sulfopropyl)-m-toluidine (TOOS), N-ethyl-N-(3-methylphenyl)-N'-acetylethylenediamine, 3-methyl-N-ethyl-N-(hydroxyethyl)aniline, N-ethyl-N-(2-hydroxy-3-sulfopropyl)aniline (ALOS), N-ethyl-N-(3-sulfopropyl)aniline (ALPS), N,N-dimethyl-m-anisidine, and N-ethyl-N-(2-hydroxy-3-sulfopropyl)-m-anisidine (ADOS). Other examples include N-(carboxymethylaminocarbonyl)-4,4'-bis(dimethylamino)-diphenylamine·sodium salt (DA-64), 10-(carboxymethylaminocarbonyl)-3,7-bis(dimethylamino)-phenothiazine·sodium salt (DA-67), 10-N-methylcarbamoyl-3,7-dimethylamino-10H-phenothiazine (MCDP), N,N,N',N',N'',N''-hexa-3-sulfopropyl-4,4',4''-triaminotriphenylmethane (TPM-PS), diaminobenzidine, hydroxyphenylpropionic acid, tetramethylbenzidine, and orthophenylenediamine.

The method for determining a substrate according to the present invention includes a step of treating a sample containing hemoglobin with the above anionic surfactant for reducing interference of hemoglobin, and a step of measuring hydrogen peroxide produced through reaction of an oxidase with the treated sample. In the present invention, these steps may be performed separately, or may be combined to a single step. The two steps may employ the same reaction temperature or different reaction temperatures, and the reaction temperature(s) is preferably selected from a temperature range within which the substrate measurement reagent of the present invention is in solution; e.g., 10 to 40°C.

In the method for determining a substrate according to the present invention, which can reduce, through oxidase reaction, interference of hemoglobin during measurement of a substrate, hemoglobin contained in a sample can be assayed by treating the sample with an anionic surfactant according to the present invention and then measuring absorbance at an absorption wavelength of hemoglobin. The method enables measurement of glycated hemoglobin (preferably hemoglobin A1c). Next will be described measurement of hemoglobin A1c through the method for determining a substrate according to the present invention.

Hemoglobin A1c, a non-enzymatically glycated product of hemoglobin contained in erythrocyte, indicates the average sugar level in a certain period of time, and therefore is considered an important index employed in clinical test. Usually, the ratio (%) of the amount of hemoglobin A1c to the total hemoglobin amount is used as an index. Therefore, in order to determine the ratio, the following four steps are required: (i) hemoglobin is released from erythrocyte through hemolysis to allow access to hemoglobin for measurement, (ii) the amount of hemoglobin present is determined, (iii) the amount of hemoglobin A1c present is determined (when this step is performed through an enzyme method, this step also includes a step of releasing hemoglobin-A1c-specific glycated peptide or glycated amino acid from hemoglobin A1c through use of a protease and a step of determining the glycated peptide or glycated amino acid through use of an oxidase specific thereto), and (iv) the ratio is calculated by dividing the amount of hemoglobin A1c by the total hemoglobin amount (calculation step). In the present specification, the term "determine a substrate" refers to not only determination of the amount of the substrate contained in a sample (e.g., concentration), but also determination of the ratio of the substrate to a certain standard substance (e.g., ratio in concentration) .

The anionic surfactant employed in the present invention is capable of methemoglobin formation. Therefore, the step (ii) can be performed through use of the anionic surfactant by measuring absorbance at an absorption wavelength of hemoglobin.

In the step (i), a known surfactant (e.g., a nonionic surfactant Triton X-100) may be employed. The anionic surfactant employed in the present invention, especially an anionic surfactant selected from among polyoxyethylene alkyl ether sulfate salts, polyoxyethylene alkyl ether phosphate esters, and triethanolamine lauryl sulfate, alkyl sulfosuccinates, and alkylphenyl ether sulfonate may be employed in the step (i). In the present invention, the anionic surfactant may be employed singly or in combination with a known surfactant. Examples of the nonionic surfactant which may be employed singly or in combination with the anionic surfactant of the present invention in the step (i) include, in addition to those known, polyoxyethylene derivative Emulgens (products of Kao Corporation, Emulgen 709, Emulgen 108, Emulgen A90, Emulgen B66, etc.), Nikkols (products of Nikko Chemicals, Nikkol BC20TX, Nikkol OP-10, Nikkol BT9, etc.), Liponoxs (products of Lion Corporation, Liponox NC80, Liponox OC100, etc.), Leocols (products of Lion Corporation, Leocol TD90, Leocol SC120, etc.), Noigens (products of Dai-ichi Kogyo Seiyaku Co., Ltd., Noigen EA120, Noigen ET147, etc.), Epans (products of Dai-ichi Kogyo Seiyaku Co., Ltd., Epan 485, Epan U103, etc.), Pluronics (products of Asahi Denka Co., Ltd., Pluronic F, Pluronic TR704, etc.), and Adekatols (products of Asahi Denka Co., Ltd., Adekatol S0120, etc.).

The step (iii) may be performed by releasing glycated peptide or glycated amino acid derived from hemoglobin A1c, from hemoglobin A1c through use of a protease and then causing a fructosyl peptide oxidase or fructosyl amino acid oxidase to act on the free glycated peptide or glycated amino acid. This step (iii) will be described in more detail. In measurement of hemoglobin A1c through use of an oxidase, the specificity of the oxidase is based on the difference in reactivity of the oxidase with fructosyl valine or fructosyl valylhistidine which respectively corresponds to valine and valylhistidine glycated at the amino terminal of hemoglobin β-subunit. A hemoglobin molecule has 44 lysine residues. Therefore, even when the reactivity of an oxidase with ε-fructosyl lysine is low, total effect of the ε-fructosyl lysine residues cannot be ignored. In addition, valine at the hemoglobin α-chain N terminus also forms fructosyl valine through glycation but does not form fructosyl valylhistidine, since the amino acid residue adjacent to the terminal valine is not histidine. Therefore, in order to measure specifically hemoglobin A1c through an enzyme method, the effect of fructosyl lysine is eliminated to the greatest possible extent, and, more advantageously, fructosyl valyl histidyl peptide rather than only fructosyl valine is measured. Therefore, from the viewpoint of obtaining improved specificity, most preferably, there are employed a protease which is capable of releasing fructosyl valyl histidyl peptide, preferably free fructosyl valylhistidine, and a fructosyl peptide oxidase which acts on fructosyl valylhistidine.

Preferably, the protease is derived from a microorganism belonging to the genus *Bacillus, Aspergillus,* or *Streptomyces,* or their recombinant products. Examples of an enzyme derived from a microorganism belonging to the genus *Bacillus* include Protin PC10F, Protin NC25 (products of Daiwa Kasei K.K.), and Toyozyme NEP (product of Toyobo, Ltd.) etc. Examples of an enzyme derived from a microorganism belonging to the genus *Aspergillus* include Molsin (product of Kikkoman Corporation). Examples of an enzyme derived from a microorganism belonging to the genus *Streptomyces* include Actinase AS, Actinase AF, Actinase E (Kaken Pharmaceutical Co., Ltd.), and protease Type-XIV (product of Sigma) etc. The enzyme may be used singly. Toyozyme NEP etc. may be used in combination with proteinase K. The enzyme preferably belongs to metalloproteinase, neutral protease, acidic protease, or basic protease. The concentration and conditions are as described above.

Examples of the fructosyl peptide oxidase described above include an enzyme which is produced through modification of a fructosyl amino acid oxidase produced from a bacterium belonging to the genus *Corynebacterium* (JP-A-2001-95598), and a fructosyl peptide oxidase derived from a filamentous fungus (JP-A-2003-235585). FPOX-CE and FPOX-EE (products of Kikkoman Corporation) are particularly preferred.

Steps (i) to (iv) may be performed sequentially, or some of the steps may be performed simultaneously. For example, in order to perform the steps (i) and (ii) simultaneously, whole blood and hemocytes may be treated with a reagent containing Triton X-100 etc. for hemolysis and the anionic surfactant of the present invention (Plysurf A208B etc.). In addition, when a protease such as proteinase K or Actinase AS is added to the reagent, step (iii) can be partially performed simultaneously with steps (i) and (ii).

In the case where the ratio of hemoglobin A1c concentration to hemoglobin concentration (%) (hereinafter may be referred to as "hemoglobin A1c (%)") is determined through use of an automatic analyzer which is widely used in clinical tests (especially in biochemical tests) (e.g., Hitachi Model 7150 automatic analyzer) (hereinafter may be referred to as "biochemical automatic analyzer") through the method for determining hemoglobin A1c according to the present invention, operation conditions of the biochemical automatic analyzer can be set with the following advantages: (1) operation conditions for measurement of hemoglobin concentration and for measurement of hemoglobin A1c concentration are set individually, (2) a reagent for determining hemoglobin concentration can also be used as a component of a reagent for determining hemoglobin A1c concentration, (3) a sample for hemoglobin concentration measurement and a sample for hemoglobin A1c concentration measurement can be used in common, and (4) the same wavelength can be employed in hemoglobin concentration measurement and hemoglobin A1c concentration measurement. The present invention also provides a method for determining hemoglobin A1c (%) by means of a biochemical automatic analyzer, characterized in that the method has the above advantages (1) to (4).

When the ratio of hemoglobin A1c concentration to hemoglobin concentration is determined through use of the reagent of the present invention, the same reaction container may be used to determine hemoglobin and hemoglobin A1c concentrations. Alternatively, when the ratio of hemoglobin A1c concentration to hemoglobin concentration of a sample is determined by means of a biochemical automatic analyzer, components of the reagent of the present invention may be sequentially added to the sample (i.e., through a single channel method). Preferably, operation conditions (such as the amount of a reagent added) of a biochemical automatic analyzer are set individually for hemoglobin concentration measurement and hemoglobin A1c concentration measurement. The reagent of the present invention enables use of a reagent for determining hemoglobin concentration as a component of a reagent for determining hemoglobin A1c concentration, and common use of a sample for both measurements. Of the measurement conditions, the same measurement wavelength can be used for hemoglobin concentration measurement and hemoglobin A1c concentration measurement.

The reagent for determining a substrate of the present invention contains (A) an anionic surfactant selected from among polyoxyethylene alkyl ether sulfate salts, polyoxyethylene alkylphenyl ether sulfate salts, polyoxyethylene alkyl ether phosphates, polyoxyethylene alkyl sulfosuccinates, polyoxyethylene alkyl ether carboxylate salts, polyoxyethylene alkyl ether sulfonate salts, triethanolamine lauryl sulfate, alkyl sulfosuccinates, and alkylphenyl ether sulfonate salts, (B) an oxidase which acts on the substrate or a substance which is produced through enzyme reaction and serves as a substrate of the oxidase, to form hydrogen peroxide, and (C) a peroxidase and an oxidizable color producing reagent.

The sensitivity can be enhanced by producing more hydrogen peroxide from a substance produced through reaction of the oxidase described in (B) above. For example, free glycated peptide or glycated amino acid released from hemoglobin A1c are reacted with a fructosyl peptide oxidase or fructosyl amino acid oxidase to thereby produce glucosone, and a glucosone-oxidizing and decomposing enzyme is added to glucosone (JP-A-2000-333696). The glucosone-oxidizing and decomposing enzyme is preferably at least one oxidase selected from the group consisting of glucose oxidase, β-galactosidase, and pyranose oxidase. In addition, as described above, a pretreatment agent for releasing hemoglobin from erythrocyte may be employed. Furthermore, there may be incorporated into the reagent an enzyme for treating impurities contained in blood; a salt such as sodium chloride, potassium chloride, or potassium ferrocyanide; a reaction-regulating agent; a tetrazolium salt for removing effect of a reducing substance; an antibiotic serving as a preservative; sodium azide; etc.

The reagent for determining a substrate according to the present invention may be provided not only in solution, but also in a dry form or in gel form. The reagent of the present invention may be packed in a glass vial, a plastic container, etc., or may be applied to or impregnated into an insoluble carrier. Examples of the insoluble carrier include particle/sphere carriers such as latex, glass, and colloid; flat plate carriers such as semiconductor and glass; film-like carriers such as those made of paper or nitrocellulose; and filament-like carriers.

Examples

The present invention will next be described in more detail by way of Examples, which should not be construed as limiting the invention thereto.

[Examples 1 to 6] Measurement of uric acid

(1) Preparation of samples

Saline or human hemoglobin solution (1 part by volume) was added to serum (9 parts by volume), to thereby prepare hemoglobin-containing samples having hemoglobin concentrations of 0, 100, 300, and 500 mg/dL.

(2) Measurement of samples

The samples were measured by means of a Hitachi Model 7150 automatic analyzer in the following manner.

<First reagent>

Anionic surfactant

Example 1; 0.5% Emal 20C (product of Kao Corporation)
Example 2; 0.5% Hitenol NF13 (product of Dai-ichi Kogyo Seiyaku Co., Ltd.)
Example 3; 0.05% Hitenol NF15 (product of Dai-ichi Kogyo Seiyaku Co., Ltd.)
Example 4; 0.5% Plysurf A208B (product of Dai-ichi Kogyo Seiyaku Co., Ltd.)
Example 5; 0.05% Pelex CS (product of Kao Corporation)
Example 6; 0.1% Pelex SS-H (product of Kao Corporation)
Comparative Example 1; Containing no anionic surfactant
Comparative Example 2; 0.05% Latemul PS + 0.05% Pelex NBL
Comparative Example 3; 0.1% Latemul PS + 0.1% Pelex NBL 500$\mu$mol/L TOOS (product of Dojindo Laboratories)
50mmol/L Phosphate buffer (pH 7.0)

(A known method (JP-A-1996-89288) for preventing interference of hemoglobin employs a combination of anionic surfactants; i.e., an alkylsulfonic acid sodium salt (trade name: Latemul PS: product of Kao Corporation) and an alkylnaphthalene sulfonic acid sodium salt (trade name: Pelex NB-L: products of Kao Corporation). The combination of the anionic surfactants employed was as described in Comparative Examples 2 and 3.)

<Second reagent>

2U/mL Uricase (product of Toyobo, Ltd.)
10U/mL Peroxidase (III) (product of Toyobo, Ltd.)
1mmol/L 4-Aminoantipyrine
50mmo1/L Phosphate buffer (pH 7.0)

Each of the first reagents (260 μL) was added to each of the samples (7 μL), the mixture was treated for 5 minutes at 37°C, and absorbance of the mixture was measured (Absorbance I). Thereafter, the second reagent (130 μL) was added to the mixture, the resultant mixture was treated for 5 minutes at 37°C, and absorbance of the mixture was measured (absorbance II). Absorbance was measured at the main wavelength of 546 nm (subwavelength of 800 nm). A control (sample blank) was prepared by use of saline instead of the sample through a same treatment. Change in absorbance of the sample was calculated from Absorbance I and Absorbance II by the following equation A. The change in absorbance was compared with that of a uric acid solution having a known concentration (20 mg/dL) which had been treated similarly, whereby concentration of uric acid in the sample was determined.

$$\text{Equation A: Absorbance = Absorbance II} - (\text{Absorbance I} \times (7+260)/(7+260+130))$$

The obtained uric acid concentration value was compared with that of the sample having a hemoglobin concentration of 0 mg/dL (100%). The results are shown in Table 1.
[Table 1]

|  | Anionic surfactants | Hemoglobin concentration (mg/dL) | | | |
|---|---|---|---|---|---|
|  |  | 0 | 100 | 300 | 500 |
| Comp. Ex. 1 | Not added | 100.0 | 92.1 | 74.8 | 77.2 |
| Comp. Ex. 2 | 0.05% Latemul PS + 0.05% Pelex NBL | 100.0 | 83.6 | 62.1 | 54.3 |
| Comp. Ex. 3 | 0.1% Latemul PS + 0.1% Pelex NBL | 100.0 | 82.4 | 72.6 | 74.0 |
| Ex. 1 | 0.5% Emal 20C | 100.0 | 95.0 | 89.0 | 85.3 |
| Ex. 2 | 0.5% Hitenol NF13 | 100.0 | 93.9 | 87.9 | 84.6 |
| Ex. 3 | 0.05% Hitenol NF15 | 100.0 | 89.8 | 86.6 | 83.6 |
| Ex. 4 | 0.5% Plysurf A208B | 100.0 | 95.5 | 94.4 | 86.4 |
| Ex. 5 | 0.05% Pelex CS | 100.0 | 92.9 | 88.9 | 88.7 |
| Ex. 6 | 0.1% Pelex SS-H | 100.0 | 97.2 | 80.5 | 78.7 |
|  |  | (%) | (%) | (%) | (%) |

As is clear from Table 1, in Comparative Examples 2 and 3 employing a combination of known anionic surfactants, interference of hemoglobin was increased as compared with the case where no surfactant was added (Comparative Example 1). In contrast, in any of Examples 1 to 6 employing an anionic surfactant according to the present invention, interference of hemoglobin was reduced as compared with the case where no surfactant was added (Comparative Example 1). This reveals that the method for determining a substrate according to the present invention is effective, even when a known method is not effective.

[Example 7] Measurement of fructosyl amino acid

(1) Preparation of samples

Hemoglobin was diluted with saline so that absorbance at 542 nm of the resultant solution was 5 OD. Through use of the hemoglobin-saline diluted solution, hemoglobin-containing samples containing fructosyl valine (fV) at concentrations of 5 μmol/L and 10 μmol/L were prepared. A control was prepared using saline instead of the hemoglobin-saline diluted solution. The fV was obtained from BioQuest.

(2) Measurement of samples
The samples were measured by means of a Hitachi Model 7150 automatic analyzer in the following manner.

<First reagent>

Anionic surfactant

Example 7; 0.2% Plysurf A208B (product of Dai-ichi Kogyo Seiyaku Co., Ltd.)
Comparative Example 4; Containing no anionic surfactant 20mmol/L Phosphate buffer (pH 8.0)

<Second reagent>

15U/mL Fructosyl amino acid oxidase (product of Kikkoman Corporation)
20U/mL Peroxidase (III) (product of Toyobo, Ltd.)
80μmol/L TPM-PS (product of Dojindo Laboratories)
200mmol/L Phosphate buffer (pH 7.0)

Each 240 μL of the first reagents was added to each 20 μL of the samples, the mixture was treated for 5 minutes at 37°C, and absorbance of the mixture was measured (Absorbance I). Thereafter, the second reagent (80 μL) was added to the mixture, the resultant mixture was treated for 5 minutes at 37°C, and absorbance of the mixture was measured (absorbance II). Measurement of absorbance and calculation of change in absorbance were carried out in a manner employed in Examples 1 to 6. The results are shown in Table 2.
[Table 2]

| fV concentration | | a | b | b/a |
|---|---|---|---|---|
| 5 | Ex. 7 | 71 | 67 | 94 |
| | Comp. Ex. 4 | 60 | 51 | 85 |
| 10 | Ex. 7 | 118 | 110 | 93 |
| | Comp. Ex. 4 | 103 | 85 | 83 |
| (μmol/L) | | (mOD/mL) | (mOD/mL) | (%) |

a: Change in absorbance of control, b: Change in absorbance of hemoglobin-containing samples

As is clear from Table 2, use of the anionic surfactant according to the present invention reduces interference of hemoglobin.

[Example 8] Measurement of fructosyl amino acid

(1) Preparation of samples

The procedure of Example 7 was repeated, to thereby prepare hemoglobin-containing samples and control samples.

(2) Measurement of samples

The samples were measured by means of a Hitachi Model 7150 automatic analyzer. The measurement procedure was as employed in Example 7, except that the following second reagent was employed.

<Second reagent>

4U/mL Fructosyl peptide oxidase (product of Kikkoman Corporation)
20U/mL Peroxidase (III) (product of Toyobo, Ltd.)
80μmol/L TPM-PS (product of Dojindo Laboratories)
200mmol/L Phosphate buffer (pH 5.5)

[Table 3]

| fV concentration | | a | b | b/a |
|---|---|---|---|---|
| 5 | Ex. 8 | 44 | 43 | 98 |
| | Comp. Ex. 5 | 43 | 26 | 60 |
| 10 | Ex. 8 | 84 | 78 | 93 |
| | Comp. Ex. 5 | 83 | 50 | 60 |
| (μmol/L) | | (mOD/mL) | (mOD/mL) | (%) |

a: Change in absorbance of control, b: Change in absorbance of hemoglobin-containing samples

As is clear from Table 3, use of the anionic surfactant according to the present invention reduces interference of hemoglobin.

[Examples 9 to 13] Measurement of hemoglobin A1c

(1) Preparation of samples

Whole blood samples were collected from 10 subjects through a conventional method by use of a blood-collecting tube containing EDTA as an anticoagulant, and were left to stand overnight in a cold room, to thereby allow erythrocyte to precipitate. A 10μL aliquot was collected from the precipitated erythrocyte layer, and 0.1% aqueous Triton X-100 solution (300 μL) was added to the aliquot, to thereby prepare hemocyte-hemolyzed samples.

(2) Measurement of samples

The obtained samples were measured by means of a Hitachi Model 7150 automatic analyzer in the following manner.

<First reagent>

Anionic surfactant

Example 9; 0.5% Plysurf A212E (product of Dai-ichi Kogyo Seiyaku Co., Ltd.)
Example 10; 0.5% Plysurf A215C (product of Dai-ichi Kogyo Seiyaku Co., Ltd.)
Example 11; 0.2% Plysurf A208B (product of Dai-ichi Kogyo Seiyaku Co., Ltd.)
Example 12; 0.5% Nikkol SBL-4N (product of NOF Corporation)
Example 13; 3.0% Emal NC35 (product of Kao Corporation)
Comparative Example 6; Containing no anionic surfactant 1U/mL Proteinase K

0.02mol/L Phosphate buffer (pH 8.0)

<Second reagent>

    4U/mL Fructosyl peptide oxidase (FPOX-CE, product of Kikkoman Corporation)
    20U/mL Peroxidase (III) (product of Toyobo, Ltd.)
    80μmol/L TPM-PS (product of Dojindo Laboratories)
    7,500U/mL Toyozyme NEP (product of Toyobo, Ltd.)*
    37.5mmol/L NaCl
    0.2mol/L Phosphate buffer (pH 5.5)

*Toyozyme NEP employed was prepared as follows: concentrated Toyozyme NEP solution (100,000 U/mL) had been dialyzed for 4 hours at 4°C against 20mmol/L phosphate buffer (pH 5.5) containing 500mmol/L NaCl.

Each 240 μL of the first reagents was added to each 20 μL of the samples, the mixture was treated for 5 minutes at 37°C, and absorbance of the mixture was measured (Absorbance III). Thereafter, the second reagent (80 μL) was added to the mixture, the resultant mixture was treated for 5 minutes at 37°C, and absorbance of the mixture was measured (absorbance IV). Absorbance was measured at the main wavelength of 600 nm. A control (sample blank) was prepared by use of saline instead of the sample through the same treatment.

Change in absorbance (Absorbance V) of each of the samples attributed to the amount of fructosyl peptide was calculated from Absorbance III and Absorbance IV using equation B.

$$\text{Equation B: Absorbance V = Absorbance IV - (Absorbance III} \times$$

$$(20+240)/(20+240+80))$$

The above absorbance III is proportional to the total amount of hemoglobin contained in a sample. Absorbance III and Absorbance V of the sample were compared with those obtained through the above procedure from a hemocyte-hemolyzed solution having a known hemoglobin A1c value (%) (hemoglobin Alc: 8.6%), to thereby calculate hemoglobin A1c value (%) of the sample.

The hemoglobin A1c values (%) obtained in Examples 9 to 13 and Comparative Example 6 were compared with that obtained through use of a commercially available kit "Rapidia A1c" (product of Fujirebio Inc.) (referential method). The results are shown in Table 4.

[Table 4]

| Sample No. | Ex. 9 | Ex. 10 | Ex. 11 | Ex. 12 | Ex. 13 | Comp. Ex. 6 | Ref. Ex. |
|---|---|---|---|---|---|---|---|
| 1 | 5.0 | 4.8 | 4.7 | 5.6 | 5.2 | -165.6 | 5.6 |
| 2 | 3.9 | 4.4 | 5.0 | 3.8 | 4.3 | -77.8 | 4.9 |
| 3 | 3.5 | 3.6 | 3.0 | 3.8 | 3.3 | -94.8 | 4.6 |
| 4 | 5.2 | 5.5 | 5.7 | 4.6 | 5.1 | -8.0 | 6 |
| 5 | 6.4 | 7.0 | 7.8 | 6.3 | 6.7 | 19.3 | 6.8 |
| 6 | 7.3 | 7.9 | 7.4 | 8.5 | 9.9 | -10.7 | 7.9 |
| 7 | 7.9 | 8.3 | 8.2 | 9.1 | 9.6 | -15.4 | 8.4 |
| 8 | 8.5 | 8.9 | 8.9 | 9.4 | 10.1 | 25.2 | 8.8 |
| 9 | 9.1 | 9.7 | 9.9 | 10.5 | 10.5 | -1.0 | 9.6 |
| 10 | 10.8 | 11.1 | 12.3 | 12.0 | 11.7 | 30.0 | 11.5 |
| Correlation Coefficient | 0.99 | 0.99 | 0.97 | 0.98 | 0.96 | 0.71 | |

Unit of values other than correlation coefficient is (%)

As is clear from Table 4, in Comparative Example 6 which employs no anionic surfactant according to the present invention, unrealistic negative hemoglobin A1c values (%) were obtained, indicating that measurement of hemoglobin A1c values (%) has failed. In contrast, hemoglobin A1c values (%) obtained through the method for determining a substrate according to the present invention exhibit good correlation with those of the referential method. These results show that the method for determining a substrate according to the present invention enables measurement of the amount of hemoglobin A1c and the total amount of hemoglobin contained in a hemoglobin-containing sample while interference of hemoglobin contained in the sample is prevented.

## Claims

1. A method for determining a substrate contained in a hemoglobin-containing sample through reaction of an oxidase with the substrate and optical measurement of the produced hydrogen peroxide by use of a peroxidase and an oxidizable color producing reagent, **characterized in that** the hemoglobin-containing sample is treated with an anionic surfactant selected from among a polyoxyethylene alkyl ether sulfate salt, a polyoxyethylene alkylphenyl ether sulfate salt, a polyoxyethylene alkyl ether phosphate, a polyoxyethylene alkyl sulfosuccinate, a polyoxyethylene alkyl ether carboxylate salt, a polyoxyethylene alkyl ether sulfonate salt, triethanolamine lauryl sulfate, an alkyl sulfosuccinate, and an alkylphenyl ether sulfonate salt.

2. A method according to Claim 1, wherein the anionic surfactant is a polyoxyethylene alkyl ether phosphate, a polyoxyethylene alkyl ether sulfate salt, or an alkyl sulfosuccinate.

3. A method according to Claim 1 or 2, wherein the substrate is uric acid, and the oxidase is uricase.

4. A method according to Claim 1 or 2, wherein the substrate is a fructosyl amino acid or a fructosyl peptide, and the oxidase is a fructosyl amino acid oxidase or a fructosyl peptide oxidase.

5. A reagent for determining a substrate contained in a hemoglobin-containing sample, containing (A) an anionic surfactant selected from among a polyoxyethylene alkyl ether sulfate salt, a polyoxyethylene alkylphenyl ether sulfate salt, a polyoxyethylene alkyl ether phosphate, a polyoxyethylene alkyl sulfosuccinate, a polyoxyethylene alkyl ether carboxylate salt, a polyoxyethylene alkyl ether sulfonate salt, triethanolamine lauryl sulfate, an alkyl sulfosuccinate, and an alkylphenyl ether sulfonate salt, (B) an oxidase which acts on the substrate to produce hydrogen peroxide, and (C) a peroxidase and an oxidizable color producing reagent.

6. A method for determining glycated protein concentration, or glycated peptide or glycated amino acid concentration, or ratio of glycated peptide or glycated amino acid concentration to glycated protein concentration, **characterized by** employing at least (1) a surfactant, (2) a protease which acts on glycated protein to release fructosyl peptide, and (3) an enzyme which acts on fructosyl peptide to produce hydrogen peroxide.

7. A method according to claim 6, wherein the surfactant is a nonionic surfactant and/or an anionic surfactant selected from among a polyoxyethylene derivative, a polyoxyethylene alkyl ether sulfate salt, a polyoxyethylene alkyl ether phosphate ester, triethanolamine lauryl sulfate, an alkyl sulfosuccinate, and an alkylphenyl ether sulfonate.

8. A method according to claim 7, wherein the phosphate ester is a monoester or a diester of a polyoxyethylene alkyl ether phosphate, or a mixture of the monoester and diester.

9. A method according to any one of claims 6 to 8, wherein the fructosyl peptide released through reaction of a protease with a glycated protein or a glycated peptide is fructosyl valylhistidine.

10. A method according to any one of claims 6 to 9, wherein the protease is derived from a microorganism belonging to the genus *Bacillus, Aspergillus,* or *Streptomyces* or their recombinant products, and releases at least fructosyl valylhistidine when one or more species of the protease are caused to act on glycated protein.

11. A method according to any one of claims 6 to 10, wherein at least fructosyl valylhistidine is a substrate of the enzyme which produces hydrogen peroxide through reaction with a fructosyl peptide.

12. A method according to any one of claims 6 to 11, wherein the glycated protein is hemoglobin A1c.

13. A method for determining hemoglobin concentration, hemoglobin A1c concentration, or ratio of hemoglobin A1c concentration to hemoglobin concentration of a hemoglobin-containing sample, **characterized by** comprising at least determining hemoglobin contained in the sample which has been treated with a surfactant, reacting a protease which releases fructosyl valylhistidine with the reaction mixture employed in the hemoglobin measurement, and determining hemoglobin A1c concentration of the mixture.

14. A method for determining hemoglobin concentration, hemoglobin A1c concentration, or ratio of hemoglobin A1c concentration to hemoglobin concentration of a hemoglobin-containing sample, **characterized by** comprising a step of mixing the sample containing hemocytes with a reaction mixture containing a surfactant to thereby release hemoglobin from the hemocytes through hemolysis, a step of diluting the resultant mixture and optically determining hemoglobin concentration of the diluted mixture, a step of causing a protease to act on hemoglobin contained in the mixture to thereby release at least fructosyl valylhistidine, a step of causing an enzyme which reacts with fructosyl valylhistidine to thereby produce hydrogen peroxide to act at least on the released fructosyl valylhistidine, a step of reacting the produced hydrogen peroxide with a peroxidase and an oxidizable color producing reagent, a step of measuring change in absorbance of the colored compound to thereby determine the hemoglobin A1c concentration, and a step of calculating ratio of hemoglobin A1c concentration to hemoglobin concentration of the sample by use of the hemoglobin concentration and the hemoglobin A1c concentration.

15. A method according to claim 14, wherein the substrate contained in a sample is fructosyl valylhistidine or a biological component other than fructosyl valylhistidine, the component being derived from the sample which is added to the reaction mixture and producing fructosyl valylhistidine through reaction with a protease, and the enzyme which produce hydrogen peroxide is a fructosyl peptide oxidase.

16. A method for pretreating a sample in measurement of hemoglobin concentration, hemoglobin A1c concentration, or ratio of hemoglobin A1c concentration to hemoglobin concentration, **characterized by** employing a nonionic surfactant and/or an anionic surfactant selected from among a polyoxyethylene derivative, a polyoxyethylene alkyl ether sulfate salt, a polyoxyethylene alkyl ether phosphate ester, triethanolamine lauryl sulfate, an alkyl sulfosuccinate, and an alkylphenyl ether sulfonate.

17. A method for determining hemoglobin concentration of a sample including the steps of releasing hemoglobin from hemocytes through hemolysis and determining hemoglobin concentration, **characterized by** employing at least a nonionic surfactant and/or an anionic surfactant selected from among a polyoxyethylene derivative, a polyoxyethylene alkyl ether sulfate salt, a polyoxyethylene alkyl ether phosphate ester, triethanolamine lauryl sulfate, an alkyl sulfosuccinate, and an alkylphenyl ether sulfonate.

18. A method for determining ratio of hemoglobin A1c concentration to hemoglobin concentration of a sample by means of a biochemical automatic analyzer, **characterized in that**, in setting operation conditions of the biochemical automatic analyzer, (1) operation conditions for measurement of hemoglobin concentration and for measurement of hemoglobin A1c concentration are individually set, (2) a reagent for determining hemoglobin concentration can also be used as a component of a reagent for determining hemoglobin A1c concentration, (3) a sample for hemoglobin concentration measurement and a sample for hemoglobin A1c concentration measurement can be used in common, and (4) the same wavelength can be employed in hemoglobin concentration measurement and hemoglobin A1c concentration measurement.

## INTERNATIONAL SEARCH REPORT

| International application No. |
|---|
| PCT/JP2004/017196 |

**A. CLASSIFICATION OF SUBJECT MATTER**
Int.Cl$^7$ C12Q1/26, 1/37, G01N33/72

According to International Patent Classification (IPC) or to both national classification and IPC

**B. FIELDS SEARCHED**

Minimum documentation searched (classification system followed by classification symbols)
Int.Cl$^7$ C12Q1/00-70, G01N33/72

Documentation searched other than minimum documentation to the extent that such documents are included in the fields searched

Electronic data base consulted during the international search (name of data base and, where practicable, search terms used)
JICST FILE(JOIS), EUROPAT(QUESTEL), MEDLINE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2001-204495 A (Asahi Kasei Corp.),<br>31 July, 2001 (31.07.01),<br>(Family: none) | 6,7,9-18<br>1-18 |
| X<br>Y | WO 2002/027331 A1 (Arkray, Inc.),<br>04 April, 2002 (04.04.02),<br>& EP 1329722 A1 & US 2004/0063213 A1 | 6,7,9-18<br>1-18 |
| X | JP 2001-095598 A (Kikkoman Corp.),<br>10 April, 2001 (10.04.01),<br>& WO 2001/025475 A1 & EP 1223224 A1 | 6,7,9-18 |
| X | WO 2002/006519 A1 (Arkray, Inc.),<br>24 January, 2002 (24.01.02),<br>& EP 1304385 A1 & US 2003/0162242 A1 | 6,7,9-18 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>01 February, 2005 (01.02.05) | Date of mailing of the international search report<br>22 February, 2005 (22.02.05) |
|---|---|
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2004/017196

C (Continuation). DOCUMENTS CONSIDERED TO BE RELEVANT

| Category* | Citation of document, with indication, where appropriate, of the relevant passages | Relevant to claim No. |
|---|---|---|
| A | JP 11-508683 A (COULTER INT. CORP.), 27 July, 1999 (27.07.99), & WO 1997/001757 A1 & US 5786224 A | 1-18 |
| A | JP 01-112155 A (Daiichi Pure Chemicals Co., Ltd.), 28 April, 1989 (28.04.89), & EP 0314046 A2 & US 4933277 A | 1-18 |
| A | JP 60-168050 A (Wako Pure Chemical Industries, Ltd.), 31 August, 1985 (31.08.85), (Family: none) | 1-18 |
| A | JP 2000-342252 A (Daiichi Pure Chemicals Co., Ltd.), 12 December, 2000 (12.12.00), (Family: none) | 1-18 |
| A | JP 2000-300394 A (Daiichi Pure Chemicals Co., Ltd.), 31 October, 2000 (31.10.00), (Family: none) | 1-18 |
| A | JP 2001-057897 A (Daiichi Pure Chemicals Co., Ltd.), 06 March, 2001 (06.03.01), (Family: none) | 1-18 |

Form PCT/ISA/210 (continuation of second sheet) (January 2004)